# EUROPEAN PATENT APPLICATION

(11) **EP 1 350 488 A2**
(43) Date of publication of application: **08.10.2003**
(21) Application number: 03251867.2
(22) Date of filing: 25.03.2003
(51) Int. Cl.: A61F 2/42

(54) **Wrist prosthesis**

(30) Priority: 26.03.2002 US 106628
(71) Applicant: DePuy Orthopaedics, Inc., Warsaw, IN 46581 (US)
(72) Inventor: Ball, Robert J., Winona Lake, IN 46590 (US); Ondrla, Jeffrey M., Leesburg, IN 46538 (US); Walters, John F., Ft Wayne, IN 46814 (US)
(74) Representative: Belcher, Simon James

(57) **Abstract**

A wrist prosthesis has first, second, and third components that in one form are a radial component, a metacarpal component, and a bearing component. The bearing component is non-fixed or non-stationary relative to one of the radial component and the metacarpal component when the wrist prosthesis is assembled such as when implanted into a patient. As such, in one form a rotational joint or union is provided on the radial component and the bearing component. The bearing component is thus free to rotationally ride on the radial component, preferably 360° thereabout. Distal the radial component, the bearing component includes an articulation surface. The metacarpal component includes an articulation surface that is complementary to the articulation surface of the bearing component. In this manner, the metacarpal component correspondingly moves relative to the bearing component. The bearing component is preferably made of a polymer such as polyethylene.

## Description

This invention relates to a wrist prosthesis.

A normal human wrist may be considered as comprising three sets of bones: the distal forearm, constituting the distal portion of the radius and the ulna; the carpals, constituting eight bones divided into two rows, i.e. the proximal bones (scaphoid, lunate, triquetrum, and pisiform) and the distal bones (trapezium, trapezoid, capitate, and hamate), that are most closely associated with the motion of the wrist; and the metacarpals, constituting the distal segments (i.e. thumb and four fingers).

The wrist is commonly considered a biaxial joint, meaning that there are two principle movements of the wrist, namely an extension-flexion movement and a radial/ulnar movement. Although the wrist has no intrinsic mechanism for active supination/pronation deviation movement, it is currently thought that there is likely some degree of passive motion associated with a torsional force transmitted across the radial-carpal joint. While various wrist prosthetics have been developed and patented, they all suffer from loosening of one of the two components of the wrist prosthetic. The torsional loads which cannot be passed onto soft tissue due to the constrained design of prostheses. This may be accelerated by a "window-wiper" action of the central stem of the metacarpal component against the dorsal aspect of the middle metacarpal.

Recognition of such passive torsional forces has led to various wrist prosthetic designs that attempt to compensate for such passive torsional forces. These designs attempt to provide a more stable fixation. One type of stable fixation design that attempts to compensate for passive torsional forces adds rotational control pegs to a metacarpal component of the wrist prosthetic. Another type of stable fixation design relies on screw-type fixation of a metacarpal component. Such designs have not been well received due to the inherently weak bone stock available for the metacarpal component in typical wrist implant patients. Also, some designs fail because there is an effort to obtain greater fixation, when motion is still present.

Another manner of attempting to compensate for such torsional forces is mismatching of wrist components. Particularly, a surgeon may match small metacarpal components with larger radial components. This, however, provides a less conforming articulating surface, thus allowing for greater contact stresses.

US-5314485 and US-4307473 disclose examples of a three-part articulating geometry for a wrist prosthetic. These designs, however, have complicated mechanics, thus creating a much higher risk of failure.

The subject invention is a wrist prosthesis having a non-fixed or non-stationary bearing component. Particularly, the subject invention is a wrist prosthesis having a rotatable bearing component. More particularly, the subject invention is a wrist prosthesis having a first component, a bearing component, and a second component, the bearing component movable about an axis of one of the first and second components. In one form, the subject invention is a wrist prosthesis having a radial component, a metacarpal component, and a bearing component, the bearing component being rotatably supported on one of the radial component and the metacarpal component. The receiving component is configured to provide an articulation surface for receiving a complementary articulation surface of the other of the components. The bearing component is rotatable about an axis substantially parallel to an axis of implantation of the radial component and/or the metacarpal component.

In one form, there is provided a wrist prosthesis having a first component, a second component, and a bearing component. The first component has a first bone anchor and a first bearing surface on an end of the first bone anchor. The second component has a second bone anchor and a bulbous member on an end of the second bone anchor, the bulbous member defining a second bearing surface. The bearing component has an upper bearing surface and a lower bearing surface and is non-fixedly mounted on one of the first and second components such that the lower bearing surface of the bearing component is adjacent the respective first or second bearing surface of the first and second components, and the upper bearing surface is adjacent the other of the respective first or second bearing surface of the first and second components. An embodiment provides implantation in the distal carpals only.

In another form, there is provided a wrist prosthesis having a radial component, a metacarpal component, and a bearing component. The radial component has a radius anchor and a support on an end of the radius anchor. The metacarpal component has a metacarpal anchor and a bulbous member defining a first articulation surface on an end of the metacarpal anchor. The bearing component is mounted for rotation on the support and has a second articulation surface that is configured to receive the first articulation surface of the bulbous member.

In yet another form, there is provided a wrist prosthesis having a radial component, a metacarpal component, and a bearing component. The radial component has a mounting surface and is configured to be fixed to a radius. The metacarpal component is configured to be fixed to a metacarpal. The bearing component is mounted for rotation on the mounting surface of the radial component and is configured to receive the metacarpal component.

In a further form, there is provided a wrist prosthesis having a radial component, a metacarpal component, and a bearing component. The radial component has a radius anchor terminating at one end in a platform, with the platform having a platform pivot portion. The metacarpal component has a metacarpal anchor terminating at one end in a bulbous member with the bulbous member defining a metacarpal articulation surface. The bearing component has a first side having a bearing pivot portion, and a second side having a bearing articulation surface configured to complementarily receive the metacarpal articulation surface for articulating movement between the metacarpal component and the bearing component. The bearing component is received on the platform such that the platform pivot portion and the bearing pivot portion cooperate for rotational movement of the bearing component relative to the radial component.

In still another form, there is provided a wrist prosthesis with a radial component having: (a) means for anchoring the radial component to a radius of a patient; and (b) means for rotationally receiving a bearing component; a bearing component having: (a) means for rotationally receiving a radial component; and (b) means for articulating the bearing component with a metacarpal component; and a metacarpal component having: (a) means for anchoring the metacarpal component to a metacarpal of a patient; and (b) means for articulating the metacarpal component with a bearing component.

The subject invention provides a wrist prosthesis that reduces forces leading to a wiper action in the wrist presented by torsional loads transmitted from the radial component of the wrist prosthesis to the metacarpal component of the wrist prosthesis through use of a mobile bearing component design.

Embodiments of the invention will now be described by way of example with reference to the accompanying drawings, in which:
Fig 1 is an exploded perspective view of an exemplary embodiment of a wrist prosthesis in accordance with the principles of the subject invention;
Fig. 2 is an exploded perspective view of the exemplary embodiment of the wrist prosthesis of Fig. 1, from a reverse angle;
Fig. 3 is an exploded front view of the exemplary embodiment of the wrist prosthesis of Fig. 1;
Fig. 4 is an exploded right side view of the exemplary embodiment of the wrist prosthesis of Fig. 1;
Fig. 5 is a side view of an alternate embodiment of a radial component and a bearing component;
Fig. 6 is an assembled radial component and bearing component particularly illustrating possible rotational motion of the bearing component relative to the radial component;
Fig. 7 is an enlarged cross-sectional view taken along line 6-6 of Fig. 2 particularly showing cross-sectional view of the rotational axis structure (i.e. the boss and boss recess) thereof;
Fig. 8 is an enlarged cross-sectional view similar to Fig. 6 but showing an alternative rotational axis for the radial component and the bearing component;
Fig. 9 is an exploded side view of another exemplary alternative embodiment of a wrist prosthesis in accordance with the subject principles;
Fig. 10 is an exploded side view of yet another exemplary alternative embodiment of a wrist prosthesis in accordance with the subject principles; and
Fig. 11 is a plan view of the subject wrist prosthesis assembled and implanted in a wrist/hand of a patient.

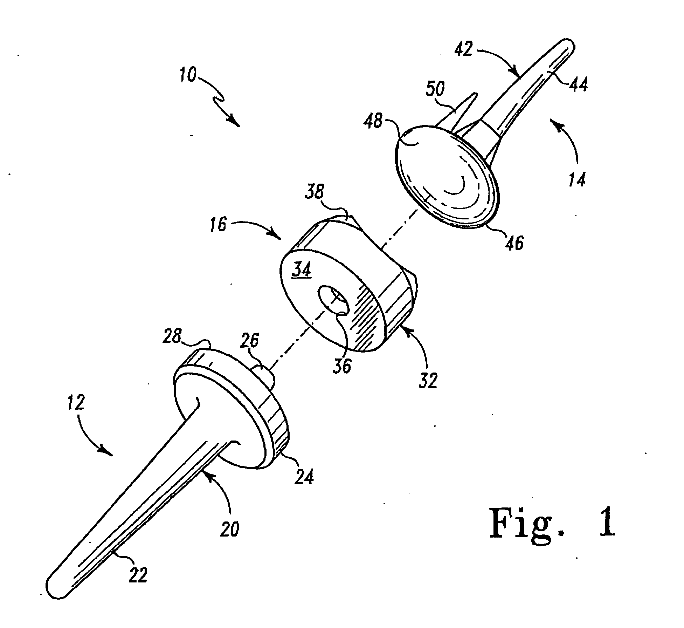

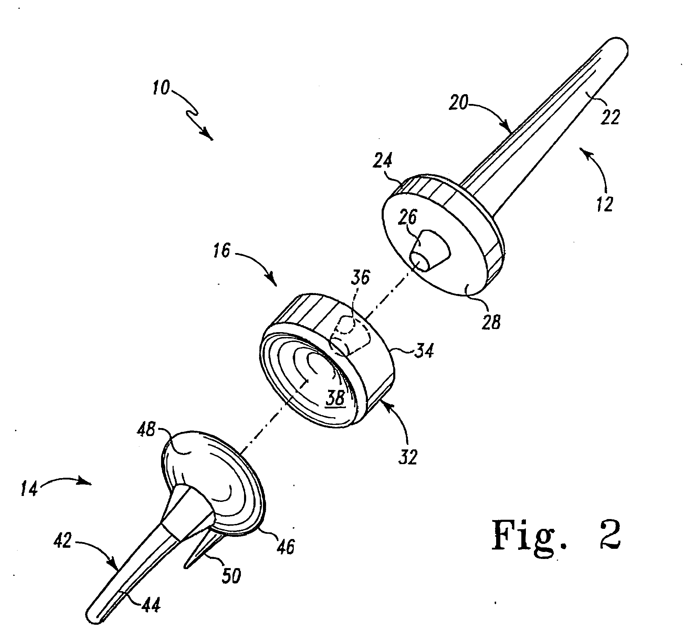

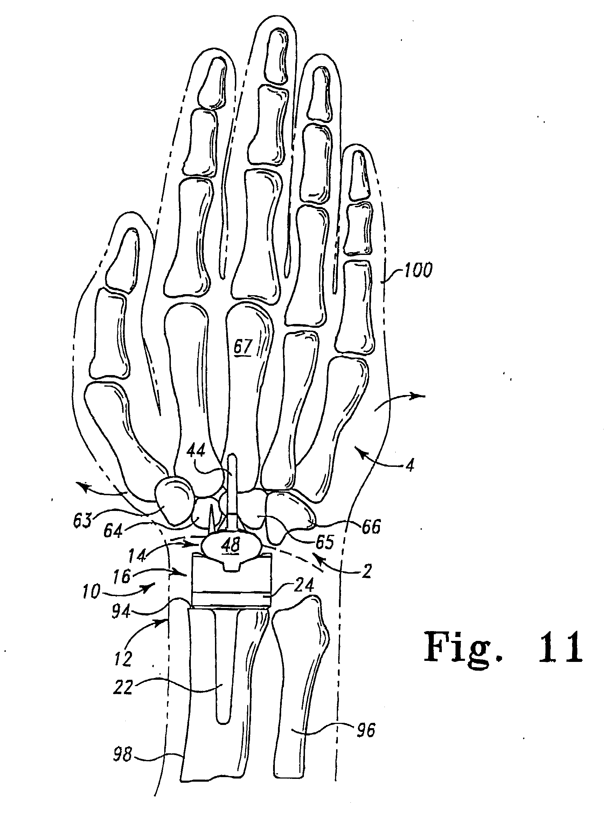

Referring to the drawings, Figs. 1-4 show a embodiment of a wrist prosthesis 10 which includes and/or may be considered as comprising a radial component or element 12, a metacarpal component or element 14, and a bearing or carpal component or element 16. The radial component 12 is configured and/or adapted to be implanted into the distal end of a radius 98 of a patient (see, e.g. Fig. 11). The metacarpal component 14 is configured to be implanted into the proximal end of a metacarpal 4 of the patient (see, e.g. Fig. 11). The bearing component 16 is configured and/or adapted to be substituted for the carpals or a portion of the carpals (such as the distal carpals) of the patient and thus be maintained between the radial component 12 and the metacarpal component 14 (see, e.g. Fig. 11). Particularly, the bearing component 16 is configured and/or adapted to be rotatably mounted to the radial component 12. More particularly, the bearing component 16 is configured and/or adapted to be maintained on the radial component 12 so as to freely rotate thereon.

The radial component 12 is characterized by a preferably one-piece body 20 preferably fabricated from a metal such as a titanium alloy or other and/or similar biocompatible metal suitable for such components. The body 20 has a stem 22 constituting and/or functioning as or being a radius anchor and/or radius anchor means. The stem 22 is configured and/or adapted to be implanted into a radius 98 of a patient (see, e.g. Fig. 11). The distal end of the stem 22 terminates in a platform, stand, support, rotational bearing surface, or the like 24. The platform 24 defines a surface 28 that is distal the stem 22. The surface 28 provides and/or functions as a rotation or rotational bearing surface for the bearing component 16. In one form, the platform 24 is essentially oval-shaped.

The radial component 12 also includes a rotation bearing 26 that is exemplified as a boss, spindle, protrusion, or the like. The rotation bearing or boss 26 extends from the surface 28 of the platform 24. While the boss 26 may take different forms, it is shown in Figs. 1-4 as frusto-conically shaped.

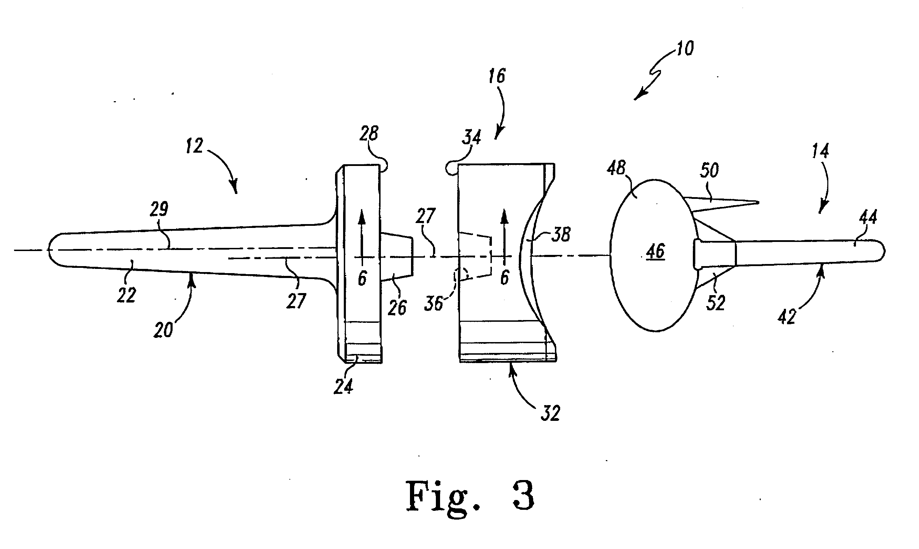

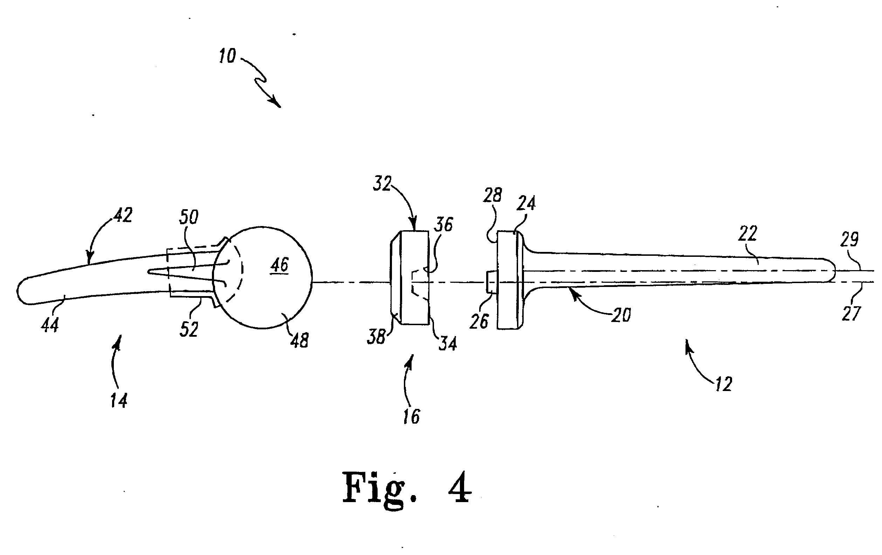

With continued reference to Figs. 3 and 4, the rotational bearing 26 defines a rotational axis represented by the line 27. Rotational movement of the bearing component 16 is about the rotational axis 27. The stem 22 includes a central axis represented by the line 29. The central axis 29 is offset from the rotational axis 27 in an ulnar direction. The rotational axis 27 (and thus the rotational bearing 26) is preferably substantially centred on the platform 24. As best seen in Fig. 11, this offset allows the bone anchor or stem 22 to be implanted into the radius while the platform or table 24 is essentially and/or substantially centred over the radius and ulnar bones.

The metacarpal component 14 is characterized by a preferably one-piece body 42 preferably fabricated from a metal such as a titanium alloy or other and/or similar biocompatible metal suitable for such components. The body 42 has a stem 44 constituting and/or functioning as or being a metacarpal anchor and/or metacarpal anchor means. The stem 44 is configured and/or adapted to be implanted into a metacarpal 4 of the patient (see, e.g. Fig. 11).

The proximal end of the stem 44 terminates in a bulbous member 46. While the bulbous member 46 may be embodied as various shapes (e.g. a sphere/spheroid, an ellipse/ellipsoid, an oval/ovoid, or other arcuate-surfaced structure), the bulbous member 46 is shown as an ellipse/ellipsoid. As such, the bulbous member 46 defines an arcuate articulation surface 48 whose curvature is defined by the particular geometry (i.e. shape) of the bulbous member 46. The metacarpal component 14 further may include a secondary metacarpal stem or anchor 50 that is configured and/or adapted to be implanted into another metacarpal 4 of the patient (see Fig. 11). A housing 52 may also be provided around the connection of the stem 44 to the bulbous member 46 if desired.

The bearing component 16 is characterized by a preferably one-piece body 32 preferably fabricated from a plastic or polymer such as polyethylene, an ultra high molecular weight/density polyethylene, or other similar biocompatible plastic/polymer suitable for such components. The body 32 is also preferably, but not necessarily, oval-shaped, and defines a surface or side 34 that provides and/or functions as a bearing surface against the radial component 12. The bearing component 16 also includes a rotation axis 36 that is exemplified as a boss recess, spindle recess, protrusion recess, bore, or the like. The rotation axis or boss recess 36 extends into the body 32 from the surface 34. While the boss recess 36 may take different forms, it is shown in Figs. 1-4 as frusto-conically shaped. It should be appreciated that the shape of the boss 26 and the boss recess 36 are preferably complementary in order to provide a rotational axis or pivot for the bearing component 16 relative to the radial component 12 (since the radial component 12, when implanted in a patient, is fixed). The bearing component 16 is thus rotationally or rotatably received/receivable on the radial component 12.

The body 32 also defines another side or surface 38. The surface 38 provides, is, and/or functions as an articulating surface for the bulbous member 46 of the metacarpal component 14. As such, at least part of the articulation surface 38 is complementarily arcuately shaped with respect to the arc or curvature of the bulbous member 46.

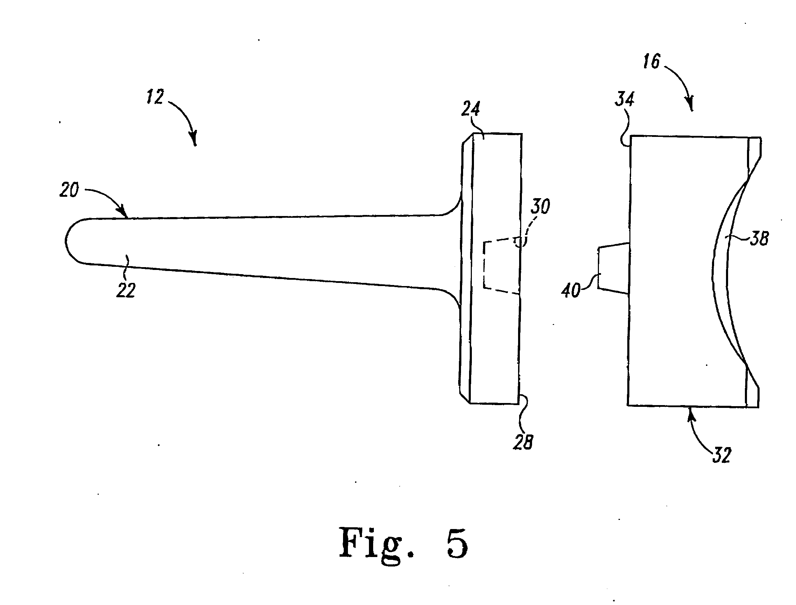

Referring to Fig. 5, there is illustrated another embodiment of the rotation axis between the radial component 12 and the bearing component 16. In this embodiment, the platform 24 of the radial component 12 includes a boss recess 30 extending therein while the side/surface 34 of the bearing component 16 has a boss 40 extending therefrom. The boss 40 is received in the boss recess 30. As such, the boss 40 and the boss recess 30 are complementary in shape and/or configuration. While not necessary, again, both the boss 40 and the boss recess 30 are frusto-conically shaped (truncated cone shaped).

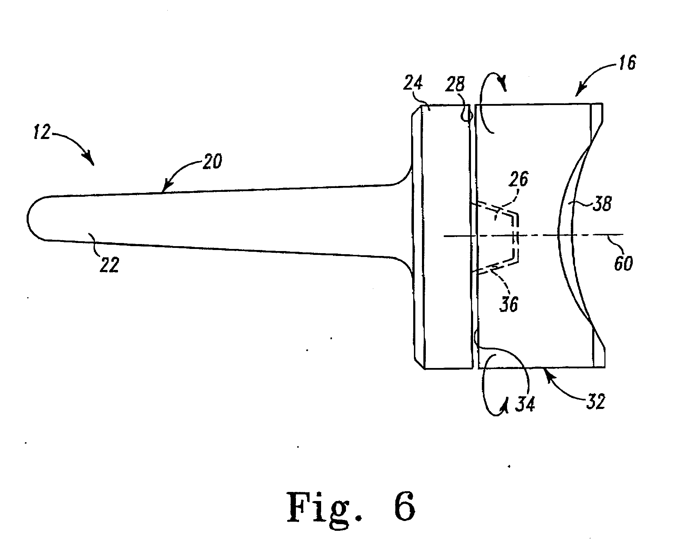

Referring to Fig. 6, the bearing component 16 is shown supported, maintained, held, or the like on and/or against the platform 24 of the radial component 12 such that the bearing component 16 may revolve, spin, rotate, and/or radially pivot with respect to the radial component 12 as represented by the arrows. The bearing component is rotatable about an axis 60 essentially defined as a centre point of the pivot defined by the pivot portions 26 and 36 of the radial component 12 and the bearing component 16 respectively. Such axis 60 may or may not be centred relative to the stem 22.

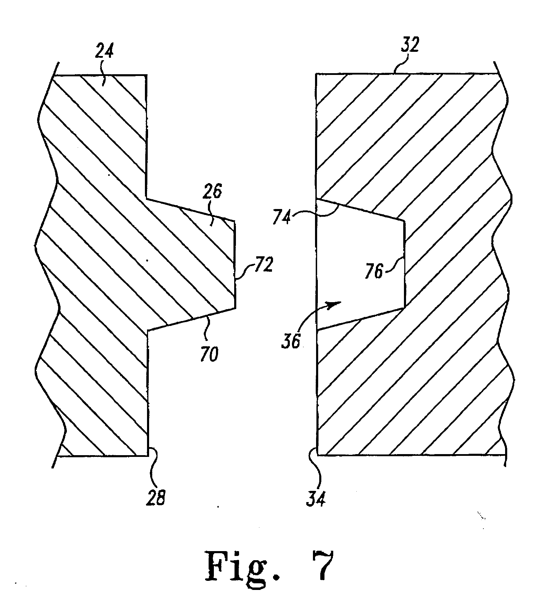

Referring to Fig. 7, the pivot between the radial component 12 and the bearing component 16 is depicted in an enlarged cross-sectional view. The boss 26 is defined in this embodiment by a frusto-conical surface 70 that terminates in a transverse end surface 72. The boss recess is defined by a frusto-conical surface 74 that terminates in a transverse end surface 76. The two frusto-conical surfaces 70 and 74 are complementary, with the boss recess surface 74 slightly larger in diameter to accommodate the boss 26. When assembled, the end surface 28 of the platform 24 abuts the end surface 34 of the body 32 such that the end surface 72 of the boss 26 preferably does not abut the end surface 76 of the boss recess 36. Additionally, the side surface 70 preferably abuts or is at least adjacent the side surface 74.

It should be appreciated that while the bearing component 16 is free to rotate 360 relative to the radial component 12, the bearing component 16 may be constrained in rotation (i.e. the degree of rotation) by the geometry of the bulbous member 46/articulation surface 48 of the metacarpal component and/or the articulation surface 38 of the bearing component 16 as well as hand/wrist properties, movement, and/or geometry. Thus, the bearing component 16 may, for example, only be able to rotate through an angle less than 360 (an arc or angle less than 360 ) due to such restriction(s). The degree of rotational movement may be mechanically restricted if necessary. However, in accordance with the principles of the subject invention, the bearing component 16 is preferably mechanically free to rotate up to 360 .

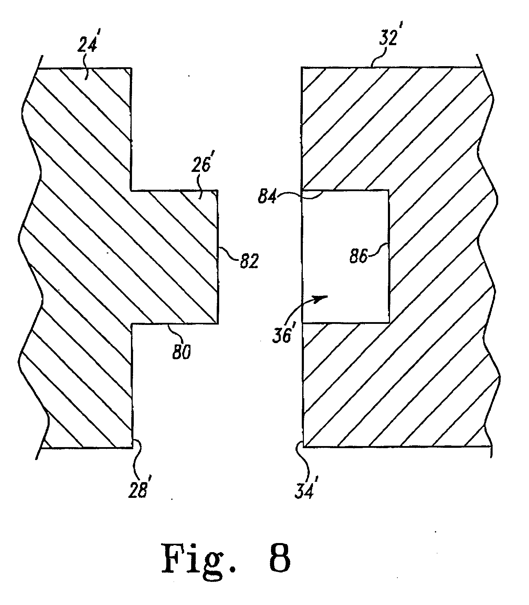

Referring now to Fig. 8, there is depicted an alternative embodiment of the cooperating rotation or pivot structures of the radial component 12 and the bearing component 16. In Fig. 8, like components to Fig. 7 are labelled with a prime adjacent the corresponding number. The platform 24' of the radial component includes a cylindrical boss 26' extending therefrom having a cylindrical side or sidewall 80 that terminates in an end 82. The bearing component body 32' includes a cylindrical boss recess 36' extending therein. The cylindrical boss recess 36' is defined by a cylindrical side or sidewall 84 and an end 86. The boss recess 36' is sized to receive the boss 26'. When so received, the bearing component body 32' is rotatably mobile on and/or relative to the platform 24' (i.e. the radial component). When assembled, the end surface 28' of the platform 24' abuts the end surface 34' of the body 32' such that the end surface 82 of the boss 26' preferably does not abut the end surface 86 of the boss recess 36'. Additionally, the side surface 80 preferably abuts or is at least adjacent the side surface 84.

It should be appreciated that the length of the boss 26' and the corresponding boss recess 36' may be varied as necessary in like manner to the boss 26 and the corresponding boss recess 36. Likewise, the boss and boss recess may take other forms such as different geometries, different number of bosses and corresponding boss recess, and/or the like.

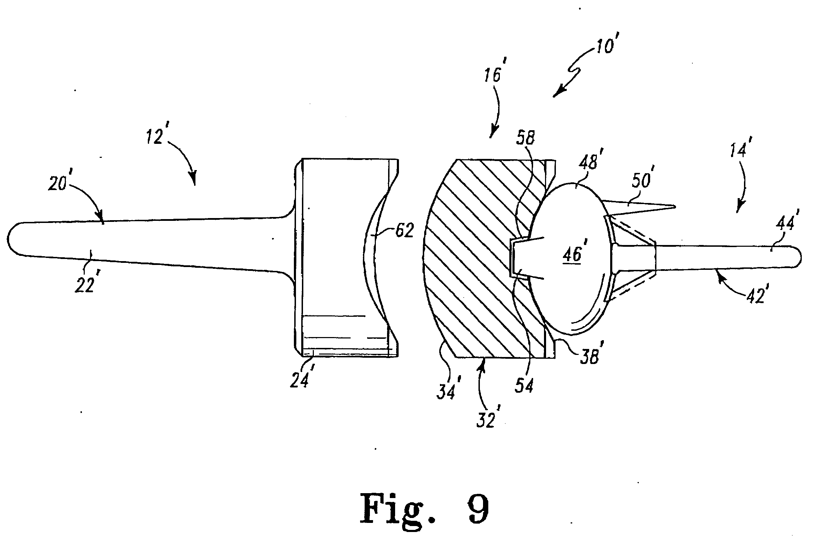

Referring to Fig. 9, there is depicted an alternate embodiment of a wrist prosthesis, generally designated 10', in accordance with the present principles. the wrist prosthesis 10' includes a first component 12', a second component 14', and a bearing component 16'. It should be appreciated that the designation of first and second for the components 12' and 14' are arbitrary and may be reversed. In Fig. 9, the first component 12' is a radial component while the second component 14' is the metacarpal component. In essence, the wrist prosthesis 10' is a reverse construct from the wrist prosthesis of Figs. 1 to 4.

The radial component 12' is configured to be implanted into a first bone, typically the radius 98 of a patient (see, e.g., Fig. 11), and to support the bearing component 16'. The radial component 12' includes a body 20' formed in like manner to the body 20 of the radial component 12. The body 20' has a stem, anchor, or the like 22', and a platform, support, or the like 24'. The stem 22' is adapted to be implanted into a radius of a patient while the platform 24' provides a support for the bearing component 16. Particularly, the platform 24 includes a bearing surface 62. The bearing surface 62 is concave and arcuate-shaped in like manner to the bearing surface 38 of the bearing component 16 (see, e.g., Figs. 1 to 4).

The bearing component 16' is formed in like manner to the bearing component 16 and has a body 32'. The body 32' has an end surface 34' that is curved, convex, and/or arcuate-shaped in a manner substantially complementary to the bearing surface 62 of the platform 24'. The bearing and/or articulation surface 34' is thus received on the bearing and/or articulation surface 62 of the bearing component 16' is a non-stationary or non-fixed manner with respect to the radial component 12'.

The bearing component 16' has another end surface 38' that is configured in like manner to the bearing and/or articulation surface 38 of the bearing component 16. The end surface 38', however, includes a boss recess 58 substantially in the centre thereof. The boss recess 58 is configured in like manner to the boss recess 36 of the bearing component 16 of Figs. 1-4. The boss recess 58 is configured to receive a boss 54 on the bearing and/or articulating surface 48' of the bulbous member 46' of the body 42' of the metacarpal component 14'.

The metacarpal component 14' is made in like manner to the metacarpal component 14 of the wrist prosthesis of Figs. 1-4. As such, the metacarpal component 14' is characterized by the body 42' defining a stem or anchor 44'. The stem 44' is configured and/or adapted to be implanted into a second bone or bones such as a metacarpal or a carpal and a metacarpal. A secondary stem or anchor 50' is also provided that is adapted to be implanted into a carpal.

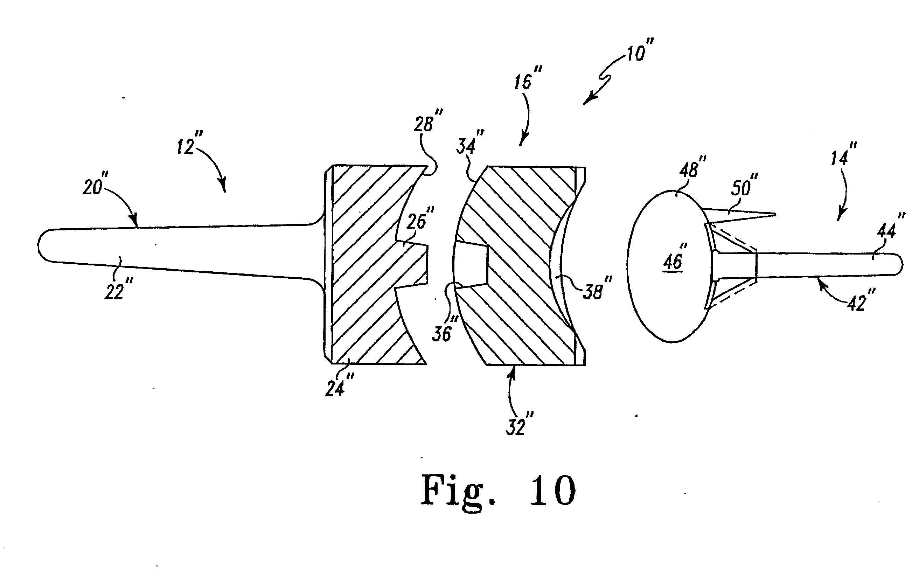

Referring to Fig. 10 there is shown another alternate embodiment of a wrist prosthesis, generally designated 10", in accordance with the present principles. In particular, in this embodiment the metacarpal component 14" is the same as the metacarpal component 14 of the wrist prosthesis 10 of Figs. 1-4. The bearing component 16" is substantially similar to the bearing component 16 of the wrist prosthesis 10 of Figs. 1-4 with the exception of the interface between the platform 24" and the end surface 34" of the bearing component body 32". This interface need not be planar as exemplified in this particular embodiment. The platform 24" includes an arcuate, curved, or concave surface 28" in either or both the A/P and the M/L aspects. The end surface 34" is arcuate, curved, or convex in either or both the A/P and the M/L aspects per the essentially complementary to the surface 28". This allows sharing of flexion-extension and R/V deviation between both articulating surfaces. The wrist prosthesis 10" provides rotation about the longitudinal axis of the stem 22' and thus the radial and motion within the sagittal and lateral plane.

A use and/or application of the subject invention will now be described. It should be appreciated, however, that the below-described use/application of the subject invention is only exemplary of one manner of use. Other manners of use not specifically described herein are contemplated. Referring to Fig. 11, there is depicted a human hand/wrist or wrist area 100 showing the bones thereof. Shown are the distal end of an ulna 96 and the distal end of a radius 98. Some, but not all, of the carpals 2 are shown, particularly shown are the trapezoid 63, trapezium 64, capitate 65, and hamate 66. As well, all of the metacarpals 4 are shown. The carpals not shown (lower row) have been resected or are removed, and are being replaced by at least a portion of the present wrist prosthesis 10.

The wrist prosthesis 10 is thus shown implanted into the hand/wrist 100. Initially, the area is prepared by resection of carpals appropriate for the circumstances, typically such as that represented by the dashed line. The radius 98 is prepared via appropriate resection to provide a seating surface 94 as is known in the art. More or less bones may be removed depending on the circumstances. Therefore it should be appreciated that the method presented herein is exemplary.

A bore corresponding to the stem 22 of the radial component 12 is first prepared in the resected radius 98. Particularly, the stem or anchor 22 of the radial component 12 is implanted and/or anchored into the radius 98. The stem 22 is anchored into the bore either with or without bone cement again, depending on circumstances. The radial component 12 is situated on the radius 98 such that the platform 24 is positioned adjacent the distal end 94 of the radius 98. In this manner, the bearing component 16 is positioned within the area in the hand/wrist 100 in which the remaining (not shown since shown as replaced) carpal bones (carpals) would be located. The bearing component 16 substitutes for one or more carpals (here, the entire proximal row of four carpals). It should be appreciated that the bearing component 32 rotates relative to the radial component 12 in all planes, constrained only in the size difference between the boss 26 and the boss recess 36. The boss 26 is, of course, smaller or less than the boss recess 36. The boss 26 may thus be described as a convex structure while the boss recess may be described as a concave structure, or vice versa. In a particular example, the bearing component 32 translates up to 20 in flexion/extension and up to 100° in radial/ulna.

The stem or anchor 44 of the metacarpal component 14 is implanted into the capitate 65, and a metacarpal bone, here shown as the middle metacarpal bone (metacarpal) 67. A bore corresponding to the stem 44 is first prepared in the capitate 65 and the metacarpal 67. The stem 44 is then anchored into the capitate 65 and the metacarpal 67 with or without bone cement as appropriate for the given circumstances. The bulbous member 48 of the metacarpal component 14 rests upon the articulate surface of the bearing component 16.

The bearing component 16 is free to rotate via the axis defined by the rotation or pivot boss/boss recess configuration as represented by the arrows in Fig. 11 relative to the radial component 12. The bearing component 16 is retained or "fixed" to the radial component 12 through soft tissue tension. Thus, twisting motion or torque of the hand is compensated for by rotation of the bearing component 16 rather than torqueing one or both of the stems 22 and 44. Translation of the bearing component 16 is restricted by the boss/boss recess configuration.

The subject invention provides various features and/or advantages. For example, the subject invention reduces torsional forces at the bone/stem and/or bone/cement interface due to rotation allowed by the subject invention. Further, since rotation is allowed within the subject wrist prosthesis 10, a more congruent articulating surface can be used while maintaining the allowance for translation and rotation between the back side of the bearing component and the radial component. The more congruent articulating surface reduces contact stress and thus presents the opportunity for longer implant life and reduced chance of subluxation. Still further, soft tissue balancing can be enhanced by the subject invention, through incorporation of differing height bearing components 16. Therefore, the height or thickness of the bearing component 16 may be variable to accommodate various differences in the geometries of the patient. It is thus contemplated that various thickness bearing components 16 may be provided and/or available. This presents the possibility of reducing the occurrence of near post-operative dislocation, currently presented as an uncommon complication. This further provides the ability of standardized post-operative care in wrist arthroplasty.

## Claims

1. A wrist prosthesis comprising:
a first component having a first bone anchor and a first bearing surface on an end of said first bone anchor;
a second component having a second bone anchor and a bulbous member on an end of said second bone anchor, said bulbous member defining a second bearing surface; and
a bearing component having an upper bearing surface and a lower bearing surface and non-fixedly mounted on one of said first and second components such that said lower bearing surface of said bearing component is adjacent the respective first or second bearing surface of the first and second components, and said upper bearing surface is adjacent the other of the respective first or second bearing surface of the first and second components.

2. The wrist prosthesis of claim 1, wherein said first component comprises one of a radial component and a metacarpal component, and said second component comprises the other of a radial component and a metacarpal component.

3. The wrist prosthesis of claim 1, wherein said bearing component is non-fixedly mounted for rotation on said one of said first and second components.

4. The wrist prosthesis of claim 3, wherein said bearing component is non-fixedly mounted for 360° rotation on and relative to said one of said first and second components.

5. The wrist prosthesis of claim 3, further comprising:
a boss on one of the one of said first and second components on which said bearing component is non-fixedly mounted for rotation and said bearing component; and
a boss recess on the other one of the one of said first and second components on which said bearing component is non-fixedly mounted for rotation and said bearing component;
said boss and said boss recess providing an axis of rotation for said bearing component.

6. A wrist prosthesis comprising:
a first component having a first bone anchor and a support on an end of said first bone anchor;
a second component having a second bone anchor and a bulbous member defining a first articulation bearing surface on an end of said second bone anchor; and
a bearing component mounted for rotation on said support, said bearing component have a second articulation surface configured to receive said first articulation surface of said bulbous member.

7. The wrist prosthesis of claim 6, wherein said bearing component is mounted for 360° rotation on said support.

8. The wrist prosthesis of claim 6, wherein said radial component and said metacarpal component are fabricated from metal, and said bearing component is fabricated from plastic.

9. The wrist prosthesis of claim 8, wherein said metal comprises a cobalt-chromium alloy, and said plastic comprises a polyethylene.

10. The wrist prosthesis of claim 9, wherein said polyethylene comprises an ultra high molecular weight polyethylene.

11. The wrist prosthesis of claim 6, further comprising:
a boss on one of said support and said bearing component; and
a boss recess on the other one of said support and said bearing component;
said boss and said boss recess providing an axis of rotation for said bearing component.

12. The wrist prosthesis of claim 11, wherein said boss and said boss recess are complementarily cylindrically shaped.

13. The wrist prosthesis of claim 11, wherein said boss and said boss recess are complementarily frustoconically-shaped.

14. The wrist prosthesis of claim 6, wherein said bulbous member comprises one of an ellipsoid, an ovoid, and a sphere.

15. A wrist prosthesis comprising:
a radial component configured to be fixed to a radius and having a mounting surface;
a metacarpal component configured to be fixed to a metacarpal; and
a bearing component mounted for rotation on said mounting surface and configured to receive said metacarpal component.

16. A wrist prosthesis comprising:
a radial component having a radius anchor terminating at one end in a platform, said platform having a platform pivot portion;
a metacarpal component having a metacarpal anchor terminating at one end in a bulbous member, said bulbous member defining a metacarpal articulation surface; and
a bearing component having a first side having a bearing pivot portion, and a second side having a bearing articulation surface configured to complementarily receive said metacarpal articulation surface for articulating movement between said metacarpal component and said bearing component, said bearing component received on said platform such that said platform pivot portion and said bearing pivot portion cooperate for rotational movement of said bearing component relative to said radial component.

17. A wrist prosthesis comprising:
a radial component having:
(a) means for anchoring said radial component to a radius of a patient; and
(b) means for rotationally receiving a bearing component;
a bearing component having:
(a) means for rotationally receiving a radial component; and
(b) means for articulating said bearing component with a metacarpal component; and
a metacarpal component having:
(a) means for anchoring said metacarpal component to a metacarpal of a patient; and
(b) means for articulating said metacarpal component with a bearing component.
